# EUROPEAN PATENT APPLICATION

(11) **EP 4 501 866 A1**
(43) Date of publication of application: **05.02.2025**
(21) Application number: 24192134.5
(22) Date of filing: 31.07.2024
(51) Int. Cl.: C02F 1/32

(54) **LIGHT SOURCE MODULE AND ULTRAVIOLET LIGHT FLUID TREATMENT DEVICE**

(30) Priority: 02.08.2023 JP 2023126554; 03.04.2024 JP 2024059955
(71) Applicant: NICHIA CORPORATION, Anan-shi Tokushima 774-8601 (JP)
(72) Inventor: IMAI, Masahiro, Anan-shi, Tokushima, 774-8601 (JP); SANO, Hiroki, Anan-shi, Tokushima, 774-8601 (JP)
(74) Representative: Winter, Brandl - Partnerschaft mbB

(57) **Abstract**

A light source module includes a substrate having an upper surface; a light source disposed on the upper surface of the substrate; and an optical member including a plurality of light controllers and disposed above the substrate with the light source interposed therebetween. The light source includes a plurality of first light emitting parts and a plurality of second light emitting parts. The plurality of light controllers include a first light controller and a plurality of second light controllers. The first light controller overlaps one or more of the plurality of first light emitting parts in a top view. Each of the plurality of second light controllers overlaps a corresponding one of the plurality of second light emitting parts in the top view.

## Description

### TECHNICAL FIELD

The present disclosure relates to a light source module and an ultraviolet light fluid treatment device.

### BACKGROUND

Light source modules including a plurality of light sources are known. For example, light source modules that include light sources arranged in a matrix on one surface of a circuit board and lens bodies disposed in front of the light sources are known. In the light source modules, each of the lens bodies is constituted by a convex lens in which one surface facing a light source is a flat surface and the other surface opposite to the one surface is an aspherical surface (see Japanese Patent Publication No. 2020-170693, for example).

### SUMMARY

It is an object of the present disclosure to provide a light source module including a novel optical member that corresponds to an arrangement of light sources.

A light source module according to an embodiment of the present disclosure includes a substrate having an upper surface; a light source disposed on the upper surface of the substrate; and an optical member including a plurality of light controllers and disposed above the substrate with the light source interposed therebetween. The light source may include a plurality of first light emitting parts and a plurality of second light emitting parts. The plurality of light controllers may include a first light controller and a plurality of second light controllers. The first light controller may overlap one or more of the plurality of first light emitting parts in a top view. Each of the plurality of second light controllers may overlap a corresponding one of the plurality of second light emitting parts in the top view.

### BRIEF DESCRIPTION OF THE DRAWINGS

A more complete appreciation of embodiments of the present disclosure and many of the attendant advantages thereof will be readily obtained by reference to the following detailed description when considered in connection with the accompanying drawings.

Other objects and further features of the present disclosure will be apparent from the following detailed description when read in conjunction with the accompanying drawings.
FIG. 1 is a perspective view of a light source module according to a first embodiment of the present disclosure.
FIG. 2 is a perspective view of the light source module illustrated in FIG. 1, from which an optical member is removed.
FIG. 3 is a top view of the light source module according to the first embodiment.
FIG. 4 is a top view of the light source module illustrated in FIG. 3, from which the optical member and wiring are removed.
FIG. 5 is a cross-sectional view of the light source module taken through line V-V of FIG. 3.
FIG. 6 is a top view of a light source module according to a first modification of the first embodiment.
FIG. 7 is a top view of the light source module illustrated in FIG. 6, from which an optical member and wiring are removed.
FIG. 8 is a perspective view of an optical member according to another example.
FIG. 9 is a top view of the optical member illustrated in FIG. 8.
FIG. 10 is a cross-sectional view of the optical member taken through line X-X of FIG. 9.
FIG. 11 is a perspective view (part 1) of a light source module according to a third modification of the first embodiment.
FIG. 12 is a side view of the light source module according to the third modification of the first embodiment.
FIG. 13 is a perspective view (part 2) of the light source module according to the third modification of the first embodiment.
FIG. 14 is a perspective view of a light source module according to a fourth modification of the first embodiment.
FIG. 15 is a top view of an optical member according to another example.
FIG. 16 is a cross-sectional view of the optical member taken through line XVI-XVI of FIG. 15.
FIG. 17 is a top view of an optical member according to another example.
FIG. 18 is a perspective view of a light source module according to a fifth modification of the first embodiment.
FIG. 19 is a perspective view of an ultraviolet light fluid treatment device according to a second embodiment.
FIG. 20 is a side view of the ultraviolet light fluid treatment device according to the second embodiment.
FIG. 21 is a cross-sectional view of the ultraviolet light fluid treatment device taken through line XXI-XXI of FIG. 19.
FIG. 22 is a cross-sectional view of the ultraviolet light fluid treatment device taken through line XXII-XXII of FIG. 20.

### DETAILED DESCRIPTION

In the following, embodiments of the present disclosure will be described with reference to the drawings. In the following description, terms indicating specific directions and positions (for example, "upper", "upward", "lower", "downward", and other terms including these terms) are used as necessary. These terms are used to facilitate understanding of the present invention with reference to the drawings, and the technical scope of the present invention is not limited by the meaning of these terms. The same reference numerals appearing in a plurality of drawings refer to the same or similar portions or members.

Further, the following embodiments exemplify a light source module and the like to embody the technical idea of the present invention, and the present invention is not limited to the following description. In addition, unless otherwise specified, the dimensions, materials, shapes, relative arrangements, and the like of components described below are not intended to limit the scope of the present invention thereto, but are described as examples. The contents described in one embodiment can be applied to other embodiments and modifications. The sizes, positional relationships, and the like of members illustrated in the drawings may be exaggerated for clearer illustration. Furthermore, in order to avoid excessive complication of the drawings, a schematic view in which some elements are not illustrated may be used, or an end view illustrating only a cut surface may be used as a cross-sectional view.

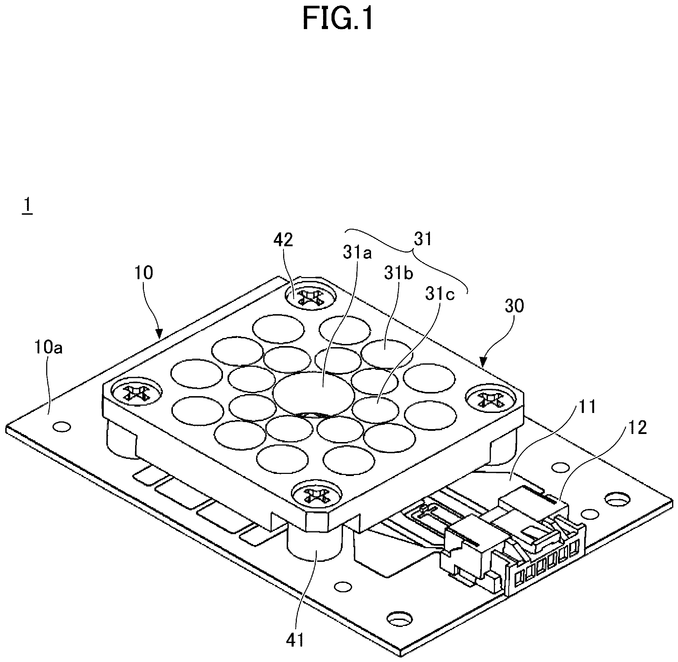

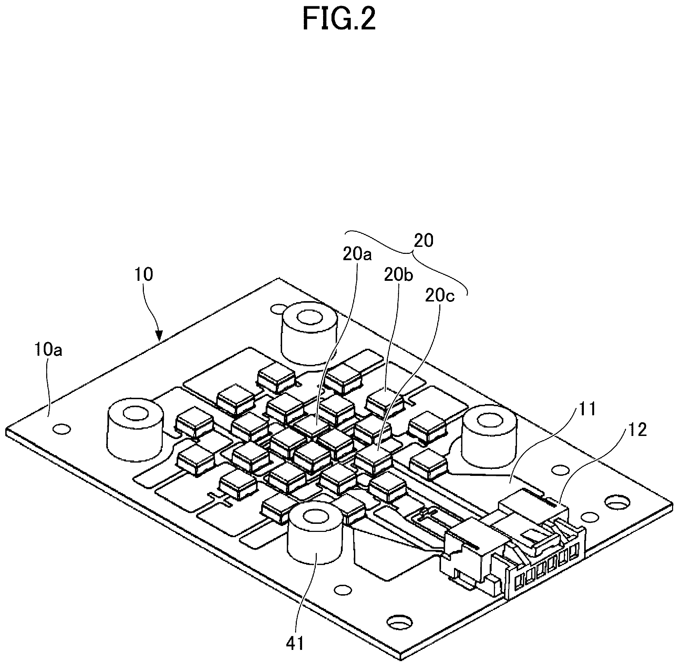

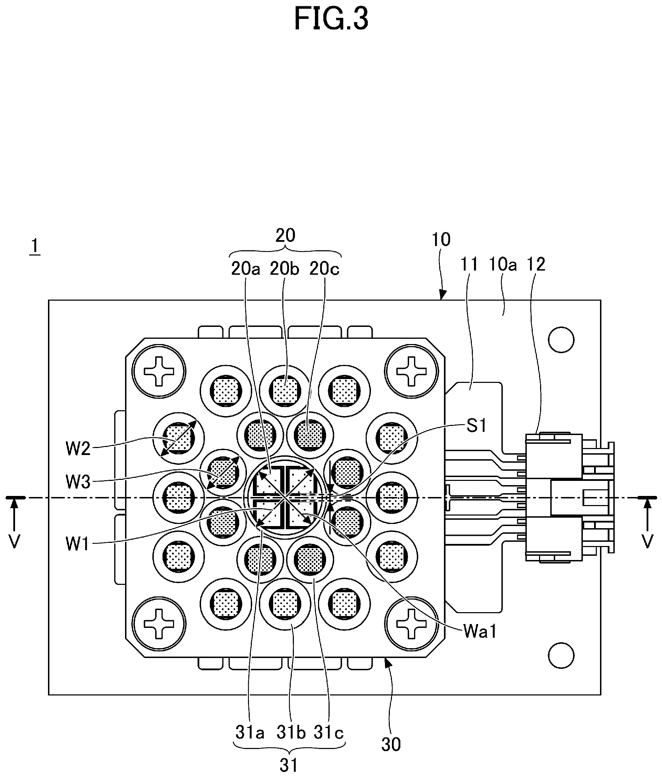

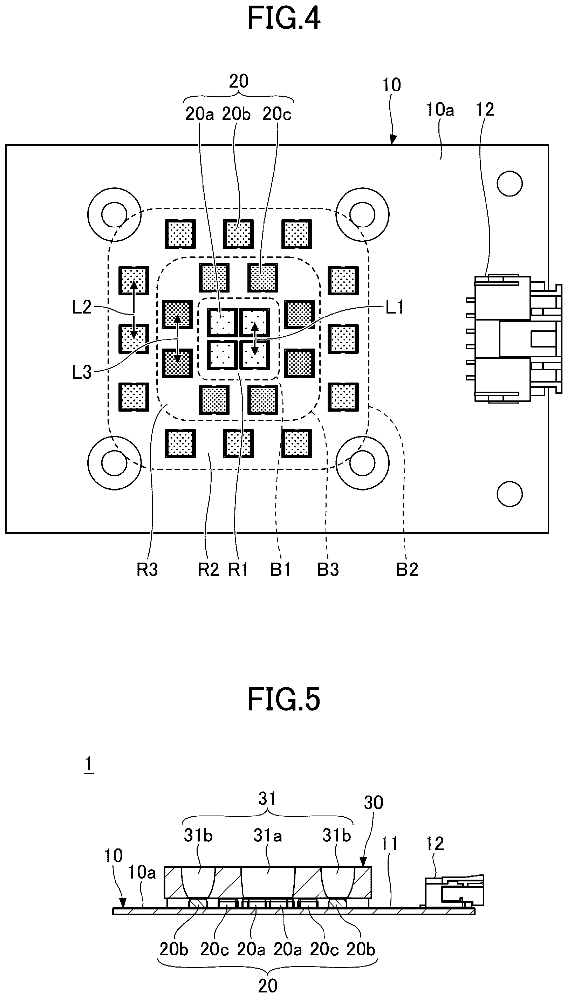

FIG. 1 is a perspective view of a light source module according to a first embodiment. FIG. 2 is a perspective view of the light source module illustrated in FIG. 1, from which an optical member is removed. FIG. 3 is a top view of the light source module according to the first embodiment. FIG. 4 is a top view of the light source module illustrated in FIG. 3, from which the optical member and wiring are removed. FIG. 5 is a cross-sectional view of the light source module taken through line V-V of FIG. 3.

As illustrated in FIG. 1 to FIG. 5, a light source module 1 includes a substrate 10, a light source 20, and an optical member 30. The light source 20 is disposed on an upper surface 10a of the substrate 10. The optical member 30 is disposed above the substrate 10 with the light source 20 interposed therebetween.

The substrate 10 includes wiring 11 having a predetermined pattern. A connector 12 electrically connected to the wiring 11 may be disposed on the upper surface 10a of the substrate 10. The substrate 10 has, as regions where the light source 20 is disposed, at least a central region R1 and a peripheral region R2 located outward of the central region R1. The central region R1 and the peripheral region R2 are regions of the upper surface 10a of the substrate 10.

In the example illustrated in FIG. 4, the substrate 10 further has an intermediate region R3 located between the central region R1 and the peripheral region R2. The central region R1 is located on the side closer to the center of the substrate 10 relative to a boundary B1. The peripheral region R2 is located between a boundary B2 and a boundary B3. The intermediate region R3 is located between the boundary B1 and the boundary B3. The boundary B1 is located inward of the boundary B2. The boundary B3 is located inward of the boundary B2 and outward of the boundary B1. In FIG. 4, the boundary B1, the boundary B2, and the boundary B3 are indicated by dashed lines, but such lines are not present in reality.

The light source 20 includes a plurality of first light emitting parts 20a and a plurality of second light emitting parts 20b. In the example illustrated in FIG. 1 to FIG. 5, the light source 20 further include a plurality of third light emitting parts 20c. In FIG. 3 and FIG. 4, for the sake of convenience, the plurality of first light emitting parts 20a are indicated by dot patterns having the same density, the plurality of second light emitting parts 20b are indicated by dot patterns having the same density, and the plurality of third light emitting parts 20c are indicated by dot patterns having the same density. The first light emitting parts 20a, the second light emitting parts 20b, and the third light emitting parts 20c are distinguished by dot patterns having different densities.

As illustrated in FIG. 4, the plurality of first light emitting parts 20a are disposed in the central region R1 in a top view. The plurality of second light emitting parts 20b are disposed in the peripheral region R2 in a top view. The plurality of third light emitting parts 20c are disposed in the intermediate region R3 in a top view. The first light emitting parts 20a, the second light emitting parts 20b, and the third light emitting parts 20c are connected in series to each other via the wiring 11. The first light emitting parts 20a are not disposed in the peripheral region R2. The second light emitting parts 20b are not disposed in the central region R1. The first light emitting parts 20a and the second light emitting parts 20b are not disposed in the intermediate region R3. The third light emitting parts 20c are not disposed in the central region R1 and the peripheral region R2.

The number of the second light emitting parts 20b disposed in the peripheral region R2 is larger than the number of the first light emitting parts 20a disposed in the central region R1. The number of the third light emitting parts 20c disposed in the intermediate region R3 is larger than the number of the first light emitting parts 20a disposed in the central region R1 and smaller than the number of the second light emitting parts 20b disposed in the peripheral region R2.

In the example illustrated in FIG. 4, the regions where the first light emitting parts 20a, the second light emitting parts 20b, and the third light emitting parts 20c are disposed are different, but the first light emitting parts 20a, the second light emitting parts 20b, and the third light emitting parts 20c are light emitting parts having the same specifications. As used herein, the term "same specifications" means that at least the wavelength range, the emission intensity, and the light distribution angle of light emitted are the same. The first light emitting parts 20a, the second light emitting parts 20b, and the third light emitting parts 20c emit, for example, ultraviolet light. In this case, the light source module 1 can emit ultraviolet light from the optical member 30. The first light emitting parts 20a, the second light emitting parts 20b, and the third light emitting parts 20c may emit light in a wavelength range other than the wavelength range of ultraviolet light.

If the first light emitting parts 20a, the second light emitting parts 20b, and the third light emitting parts 20c emit ultraviolet light, the peak wavelength of the ultraviolet light is, for example, 10 nm or more and 405 nm or less. A difference in peak wavelength of light emitted from the first light emitting parts 20a, the second light emitting parts 20b, and the third light emitting parts 20c is preferably within 5 nm. The first light emitting parts 20a, the second light emitting parts 20b, and the third light emitting parts 20c include light emitting elements. As the light emitting elements, light emitting diodes (LEDs) or laser diodes (LDs) can be used, for example. As each of the first light emitting parts 20a, the second light emitting parts 20b, and the third light emitting parts 20c, a light emitting device in which a light emitting element is disposed in a package can be used. As the material of the package, a ceramic or a resin can be used.

The first light emitting parts 20a, the second light emitting parts 20b, and the third light emitting parts 20c may emit light in different wavelength ranges. For example, a first light emitting part 20a, a second light emitting part 20b, and a third light emitting part 20c may include two or more light emitting parts from among a light emitting part having a light emission peak wavelength of 260 nm or more and 280 nm or less, a light emitting part having a light emission peak wavelength of greater than 280 nm and less than 315 nm, and a light emitting part having a light emission peak wavelength of 315 nm or more and 405 nm or less. For example, as a configuration in which the above-described three light emitting parts are included, a configuration in which the first light emitting part 20a has a light emission peak wavelength of 260 nm or more and 280 nm or less, the second light emitting part 20b has a light emission peak wavelength of 315 nm or more and 405 nm or less, and the third light emitting part 20c has a light emission peak wavelength of greater than 280 nm and less than 315 nm can be employed. Further, the above-described light emission peak wavelength of the first light emitting part 20a may be replaced with the above-described light emission peak wavelength of the second light emitting part 20b, the above-described light emission peak wavelength of the first light emitting part 20a may be replaced with the above-described light emission peak wavelength of the third light emitting part 20c, and the above-described light emission peak wavelength of the second light emitting part 20b may be replaced with the above-described light emission peak wavelength of the third light emitting part 20c.

In the example illustrated in FIG. 1 to FIG. 5, four first light emitting parts 20a are disposed in the central region R1. Further, twelve second light emitting parts 20b are disposed in the peripheral region R2. Further, eight third light emitting parts 20c are disposed in the intermediate region R3. The first light emitting parts 20a, the second light emitting parts 20b, and the third light emitting parts 20c can be disposed so as to have four-fold symmetry in a top view, for example. The number of light emitting parts disposed in each of the regions is not limited to the example illustrated in FIG. 1 to FIG. 5. Unless otherwise specified, the term "top view" refers to viewing the light source module 1 from a direction normal to the upper surface 10a of the substrate 10.

The optical member 30 is fixed to the substrate 10 with a screw 42 via a spacer 41, for example. The outer shape of the optical member 30 may be a square shape in a top view. The outer shape of the optical member 30 may be a rectangular shape, a circular shape, or an elliptical shape in a top view. As used herein, each of the square shape and the rectangular shape includes not only a shape having right-angle corners but also a shape having chamfered corners. As the material of the optical member 30, a metal or a resin can be used. Examples of the metal include aluminum and an aluminum alloy. Examples of the resin include a fluorine-based resin. A film having a high light reflectance may be formed on the surface of the metal or the resin. Examples of the film having a high light reflectance is an aluminum film.

The optical member 30 includes a plurality of light controllers 31. Each of the light controllers 31 is an opening penetrating the optical member 30 in the direction normal to the upper surface 10a of the substrate 10. In each of the light controllers 31, the opening area of the opening is the smallest on the light source 20 side and the opening area of the opening increases as the distance from the light source 20 increases. By having such a shape, each of the light controllers 31 can function as a reflector. In the example illustrated in FIG. 1 to FIG. 5, the opening has a circular shape in a top view. The opening may have an elliptical shape, a square shape, a rectangular shape, or the like in a top view.

The plurality of light controllers 31 include a first light controller 31a and a plurality of second light controllers 31b. In the example illustrated in FIG. 1 to FIG. 5, the plurality of light controllers 31 further include a plurality of third light controllers 31c. In a top view, the first light controller 31a is disposed in the central region R1 and the plurality of second light controllers 31b are disposed in the peripheral region R2. In a top view, the third light controllers 31c are disposed in the intermediate region R3.

The first light controller 31a overlaps the plurality of first light emitting parts 20a in a top view. Each of the plurality of second light controllers 31b overlaps a corresponding one of the second light emitting parts 20b in a top view. In the example illustrated in FIG. 1 to FIG. 5, the first light controller 31a overlaps four first light emitting parts 20a in a top view. Further, each of twelve second light controllers 31b overlaps a corresponding one of the second light emitting parts 20b in a top view.

Light from the plurality of first light emitting parts 20a, which overlap the first light controller 31a in a top view, is incident on the first light controller 31a, and the incident light is reflected in an opening of the first light controller 31a and is emitted from the side opposite to the substrate 10. Light from a second light emitting part 20b, which overlaps a corresponding one of the second light controllers 31b in a top view, is incident on the corresponding one of the second light controllers 31b, and the incident light is reflected in an opening of the corresponding one of the second light controllers 31b and is emitted from the side opposite to the substrate 10. Light from a third light emitting part 20c, which overlaps a corresponding one of the third light controllers 31c in a top view, is incident on the corresponding one of the third light controllers 31c, and the incident light is reflected in an opening of the corresponding one of the third light controllers 31c and is emitted from the side opposite to the substrate 10.

As described above, in the light source module 1, the first light controller 31a constituting part of the optical member 30 overlaps the plurality of first light emitting parts 20a in a top view. Further, each of the plurality of second light controllers 31b constituting part of the optical member 30 overlaps a corresponding one of the second light emitting parts 20b in a top view. Accordingly, this makes it possible to achieve the light source module including the optical member 30 in which the plurality of first light emitting parts 20a disposed in the central region R1 are optically controlled by one light controller 31 in a collective manner and the plurality of second light emitting parts 20b disposed in the peripheral region R2 are optically controlled by respective light controllers 31.

Further, the light source module 1 may include the plurality of third light emitting parts 20c and the plurality of third light controllers 31c as necessary, and each of the plurality of third light controllers 31c can be disposed so as to overlap a corresponding one of the third light emitting parts 20c in a top view. Accordingly, the light source module including the optical member 30 corresponding to the arrangement of the first light emitting parts 20a, the second light emitting parts 20b, and the third light emitting parts 20c of the light source 20 can be achieved.

In the example of the light source module 1, light having a relatively wide light distribution angle may be emitted from the first light controller 31a in the central region R1. In the peripheral region R2, light having a relatively narrow light distribution angle is preferably emitted from each of the second light controllers 31b. The light distribution angle of the light emitted from each of the second light controllers 31b is smaller than the light distribution angle of the light emitted from the first light controller 31a, and thus the light source module 1 as a whole can emit light having narrow light distribution. The light distribution angle of the light emitted from the first light controller 31a refers to an angle (full width at half maximum) at which the intensity of the light is one-half of the maximum intensity of the light when the overall intensity of the light emitted from the first light controller 31a is measured.

In the example illustrated in FIG. 1 to FIG. 5, a distance L1 between adjacent first light emitting parts 20a is smaller than a distance L2 between adjacent second light emitting parts 20b in a top view. Further, the distance L1 between the adjacent first light emitting parts 20a is smaller than a distance L3 between adjacent third light emitting parts 20c. Accordingly, the density of the arrangement of the plurality of first light emitting parts 20a can be increased. Further, the distance L2 between the adjacent second light emitting parts 20b is longer than or equal to the distance L3 between the adjacent third light emitting parts 20c. In the example illustrated in FIG. 1 to FIG. 5, the distance L2 between the adjacent second light emitting parts 20b is longer than the distance L3 between the adjacent third light emitting parts 20c. The distance between adjacent light emitting parts refers to the shortest distance between the centers of adjacent light emitting parts.

Further, in the example illustrated in FIG. 1 to FIG. 5, the interval between the adjacent first light emitting parts 20a is smaller than the interval between the adjacent second light emitting parts 20b in a top view. Further, the interval between the adjacent first light emitting parts 20a is smaller than the interval between the adjacent third light emitting parts 20c in a top view. Accordingly, the density of the arrangement of the plurality of first light emitting parts 20a can be increased. Further, the interval between the adjacent second light emitting parts 20b is greater than or equal to the interval between the adjacent third light emitting parts 20c in a top view. In the example illustrated in FIG. 1 to FIG. 5, the interval between the adjacent second light emitting parts 20b is greater than the interval between the adjacent third light emitting parts 20c.

Further, in the central region R1, the first light controller 31a overlaps the plurality of first light emitting parts 20a in a top view. Thus, the interval between first light emitting parts 20a disposed adjacent to each other in the central region R1 can be reduced as compared to when each of a plurality of light controllers 31 overlaps a corresponding first light emitting part 20a in a top view. Accordingly, the number of first light emitting parts 20a disposed in the central region R1 can be increased. Therefore, the illuminance of the entire light source module 1 can be improved.

Further, the light emitted from the first light controller 31a in the central region R1 has a has a relatively wide light distribution angle. Thus, a portion of the light emitted from the first light controller 31a overlaps the light emitted from the second light controllers 31b in the peripheral region R2. Therefore, a region having low illuminance is less likely to occur between adjacent light controllers, and thus a variation in the illuminance distribution of the entire light source module 1 can be reduced.

In the example illustrated in FIG. 1 to FIG. 5, a width W1 of the first light controller 31a is greater than a width W2 of a second light controller 31b in a top view. For example, the width W1 of the first light controller 31a is 1.5 times or more and 4.0 times or less the width W2 of the second light controller 31b. Further, the width W1 of the first light controller 31a is greater than a width W3 of a third light controller 31c in a top view. Further, the width W2 of the second light controller 31b is greater than or equal to the width W3 of the third light controller 31c in a top view. For example, the width W2 of the second light controller 31b is 1.0 times or more and 1.3 times or less the width W3 of the third light controller 31c. In the example illustrated in FIG. 1 to FIG. 5, the width W2 of the second light controller 31b is greater than the width W3 of the third light controller 31c. With this configuration, third light controllers 31c can be disposed close to each other. The width W1 of the first light controller 31a, the width W2 of the second light controller 31b, and the width W3 of the third light controller 31c are the maximum widths of respective openings. Further, in the example illustrated in FIG. 1 to FIG. 5, the minimum width of the opening of the first light controller 31a is greater than the minimum width of the opening of the second light controller 31b in a top view. Further, the minimum width of the opening of the first light controller 31a is greater than the minimum width of the opening of the third light controller 31c in a top view. Further, the minimum width of the opening of the second light controller 31b is greater than or equal to the minimum width of the opening of the third light controller 31c in a top view. In the example illustrated in FIG. 1 to FIG. 5, the minimum width of the opening of the second light controller 31b is equal to the minimum width of the opening of the third light controller 31c. The width of a light controller is the diameter if the light controller has a circular shape in a top view, the major axis if the light controller has an elliptical shape, the length of the diagonal if the light controller has a rectangular shape or a square shape, or the length of the longest straight line that can be continuously drawn in the light controller if the light controller has any other shape.

By setting the distance between light emitting parts and the widths of light controllers as described above, light having narrow light distribution is easily emitted from the peripheral region.

In the example illustrated in FIG. 1 to FIG. 5, a portion of each of the plurality of first light emitting parts 20a does not overlap the opening on the first light emitting part 20a side of the first light controller 31a in a top view. Further, a portion of a second light emitting part 20b does not overlap the opening on the second light emitting part 20b side of the second light controller 31b in a top view. Further, a portion of a third light emitting part 20c does not overlap the opening on the third light emitting part 20c side of the third light controller 31c in a top view. An opening on the light emitting part side of a light emitting part is an opening having the smallest opening area. The width on the second light emitting part 20b side of the second light controller 31b is smaller than the width of the second light emitting part 20b. The width on the second light emitting part 20b side of the second light controller 31b can be, for example, 0.5 times or more and less than 1.0 times the width of the second light emitting part 20b. The width on the third light emitting part 20c side of the third light controller 31c is smaller than the width of the third light emitting part 20c. The width on the third light emitting part 20c side of the third light controller 31c can be, for example, 0.5 times or more and less than 1.0 times the width of the third light emitting part 20c. If the outer shape of a light emitting part has a rectangular shape in a top view, the width of the light emitting part can be, for example, 3.0 mm or more and 8.0 mm or less. With this configuration, light controllers can be disposed close to each other. Accordingly, the size of the light source module can be reduced.

The plurality of first light emitting parts 20a are located within the opening on the light emission side of the first light controller 31a in a top view. Further, the second light emitting part 20b is located within the opening on the light emission side of the second light controller 31b in a top view. Further, the third light emitting part 20c is located within the opening on the light emission side of the third light controller 31c. An opening on the light emission side of a light controller is an opening having the maximum opening area. As described above, the size of an opening with respect to a light emitting part is set such that a portion of the light emitting part is not located within the opening on the light emitting part side of a light controller in a top view and the entire light emitting part is located within the opening on the light emission side of the light controller in a top view. Accordingly, the light source module in consideration of both light extraction and close arrangement of light controllers can be achieved.

In the example illustrated in FIG. 1 to FIG. 5, an interval S1 between two adjacent first light emitting parts 20a is smaller than a value that is twice a difference T between the maximum width W1 and the minimum width Wa1 of the opening of the first light controller 31a. In order to effectively control light from a plurality of first light emitting parts 20a arranged in this manner, it is preferable to provide the first light controller 31a that overlaps the plurality of first light emitting parts 20a.

In the example illustrated in FIG. 1 to FIG. 5, the interval between the first light controller 31a and a second light controller 31b located closest to the first light controller 31a is smaller than the width W3 of the third light controller 31c in the top view. With this configuration, light controllers can be arranged close to each other. Accordingly, the size of the light source module can be reduced.

### Modifications of Light Source Module

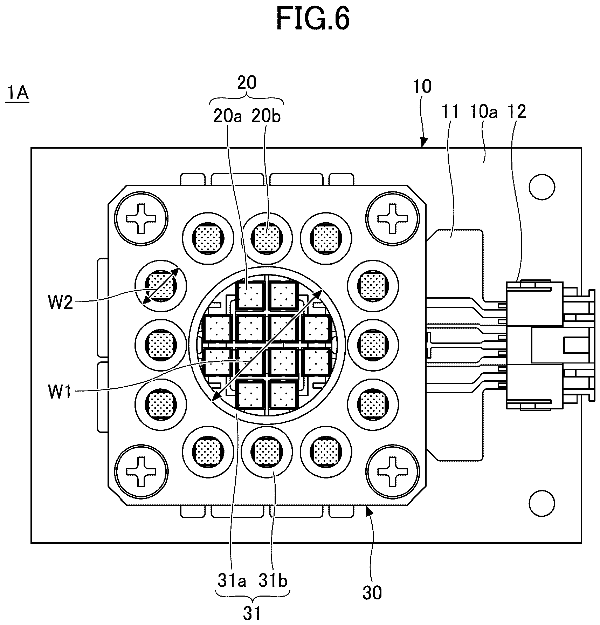

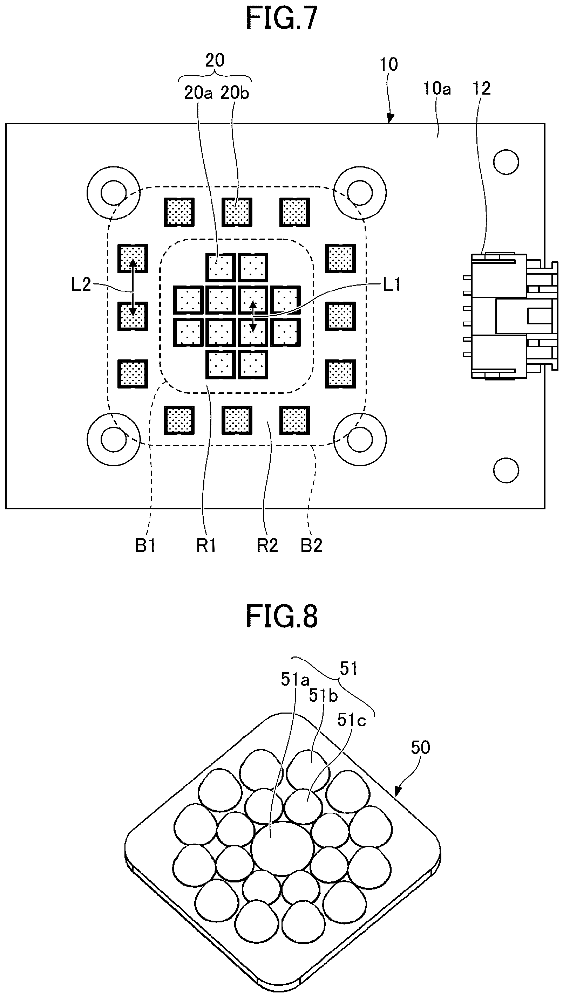

FIG. 6 is a top view of a light source module according to a first modification of the first embodiment. FIG. 7 is a top view of the light source module illustrated in FIG. 6, from which an optical member and wiring are removed. A light source module 1A illustrated in FIG. 6 and FIG. 7 differs from the light source module 1 in the arrangement of a light source 20. Further, the arrangement of light controllers 31 of an optical member 30 is also changed from that of the light source module 1 in accordance with the arrangement of the light source 20.

In the light source module 1A, a substrate 10 has a central region R1 and a peripheral region R2 as regions where the light source 20 is disposed, and does not have an intermediate region R3. The central region R1 is located on the side closer to the center of the substrate 10 relative to a boundary B1. The peripheral region R2 is located between the boundary B1 and a boundary B2. In FIG. 7, the boundary B1 and the boundary B2 are indicated by dashed lines, but such lines are not present in reality.

The light source 20 includes a plurality of first light emitting parts 20a and a plurality of second light emitting parts 20b. The plurality of first light emitting parts 20a are disposed in the central region R1. The plurality of second light emitting parts 20b are disposed in the peripheral region R2. The first light emitting parts 20a and the second light emitting parts 20b are connected in series to each other via wiring 11.

In the example illustrated in FIG. 6 and FIG. 7, twelve first light emitting parts 20a are disposed in the central region R1. Further, twelve second light emitting parts 20b are disposed in the peripheral region R2. The first light emitting parts 20a and the second light emitting parts 20b can be disposed so as to have four-fold symmetry in a top view, for example. The number of light emitting parts disposed in each of the regions is not limited to the example illustrated in FIG. 6 and FIG. 7.

A plurality of light controllers 31 include a first light controller 31a and a plurality of second light controllers 31b. The first light controller 31a overlaps the plurality of first light emitting parts 20a in a top view. Each of the plurality of second light controllers 31b overlaps a corresponding one of the second light emitting parts 20b in a top view. In the example illustrated in FIG. 6 and FIG. 7, the first light controller 31a overlaps the twelve first light emitting parts 20a in a top view.

Light from the twelve first light emitting parts 20a, which overlap the first light controller 31a in a top view, is incident on the first light controller 31a, and the incident light is reflected in an opening of the first light controller 31a and is emitted from the side opposite to the substrate 10. Light from a second light emitting part 20b, which overlaps a corresponding one of the second light controllers 31b in a top view, is incident on the corresponding one of the second light controllers 31b, and the incident light is reflected in an opening of the corresponding one of the second light controllers 31b and is emitted from the side opposite to the substrate 10.

As described above, in the light source module 1A, the first light controller 31a constituting part of the optical member 30 overlaps the plurality of first light emitting parts 20a in a top view. Further, each of the plurality of second light controllers 31b constituting part of the optical member 30 overlaps a corresponding one of the second light emitting parts 20b in a top view. Accordingly, the same effects as those of the light source module 1 can be obtained.

The relationship between a distance L1 between adjacent first light emitting parts 20a and a distance L2 between adjacent second light emitting parts 20b and the relationship between a width W1 of the first light controller 31a and a width W2 of each of the second light controllers 31b can be the same as those of the light source module 1.

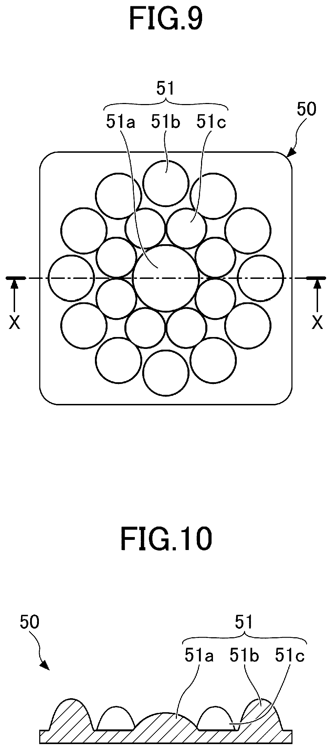

FIG. 8 is a perspective view of an optical member according to another example. FIG. 9 is a top view of the optical member illustrated in FIG. 8. FIG. 10 is a cross-sectional view of the optical member taken through line X-X of FIG. 9. The light source module 1 may include an optical member 50 instead of the optical member 30.

The optical member 50 includes a plurality of light controllers 51. In the example illustrated in FIG. 8 to FIG. 10, the optical member 50 has an upper surface and a lower surface, and the plurality of light controllers 51 are disposed on the upper surface of the optical member 50. Each of the light controllers 51 is a protrusion protruding toward the side opposite to a light source 20. The protrusion is the thickest at the center and becomes thinner as the distance from the center increases. The thickness of the protrusion refers to the distance from the upper surface of the optical member 50 to the upper surface of the protrusion in a direction normal to the upper surface of the optical member 50. Further, each of the light controllers 51 (each of protrusions) can function as a convex lens. In the example illustrated in FIG. 8 to FIG. 10, each of the protrusions has a circular shape in a top view. Each of the protrusions may have an elliptical shape, a square shape, a rectangular shape, or the like in a top view. The optical member 50 can be formed of, for example, glass or a resin. Example of the glass include quartz glass and borosilicate glass. Examples of the resin include a silicone-based resin.

The plurality of light controllers 51 include a first light controller 51a and a plurality of second light controllers 51b. In the example illustrated in FIG. 8 to FIG. 10, the plurality of light controllers 51 further includes a plurality of third light controllers 51c. The first light controller 51a is disposed in a central region in a top view of the optical member 50. The second light controllers 51b are disposed in a peripheral region in a top view of the optical member 50. The third light controllers 51c are disposed in an intermediate region between the central region and the peripheral region in a top view of the optical member 50. When the light source module 1 is assembled, the first light controller 51a overlaps a plurality of first light emitting parts 20a in a top view, and each of the plurality of second light controllers 51b overlaps a corresponding one of second light emitting parts 20b. Further, each of the plurality third light controllers 51c overlaps a corresponding one of third light emitting parts 20c.

Light from the plurality of first light emitting parts 20a, which overlap the first light controller 51a in a top view, is incident on the first light controller 51a, and the light distribution angle of the incident light is narrowed in a protrusion of the first light controller 51a and is emitted from the side opposite to the substrate 10. Light from a second light emitting part 20b, which overlaps a corresponding one of the second light controllers 51b in a top view, is incident on the corresponding one of the second light controllers 51b, and the light distribution angle of the incident light is narrowed in a protrusion of the corresponding one of the second light controllers 51b and is emitted from the side opposite to the substrate 10. Light from a third light emitting part 20c, which overlaps a corresponding one of the third light controllers 51c in a top view, is incident on the corresponding one of the third light controllers 51c, and the light distribution angle of the incident light is narrowed in a protrusion of the corresponding one of the third light controllers 51c and is emitted from the side opposite to the substrate 10. Even when the optical member 50 including the light controllers 51 each having a convex shape is used, the same effects as in the case of the optical member 30 including the light controllers 31, which are openings, can be obtained.

The height of the first light controller 51a is smaller than the height of each of the second light controllers 51b and the height of each of the third light controllers 51c. The height of each of the third light controllers 51c is smaller than or equal to the height of each of the second light controllers 51b. In the example illustrated in FIG. 10. the height of each of the third light controllers 51c is smaller than the height of each of the second light controllers 51b. With this configuration, the height of the entirety of the light controllers 51 can be reduced.

The relationship among the width of the first light controller 51a, the width of each of the second light controllers 51b, and the width of each of the third light controllers 51c can be the same as that of the optical member 30.

The optical member 50 may be applied to the light source module 1A. In this case, the first light controller 51a constituting part of the optical member 50 may overlap the plurality of first light emitting parts 20a in a top view, and each of the plurality of second light controllers 51b constituting part of the optical member 50 may overlap a corresponding one of the second light emitting parts 20b in a top view.

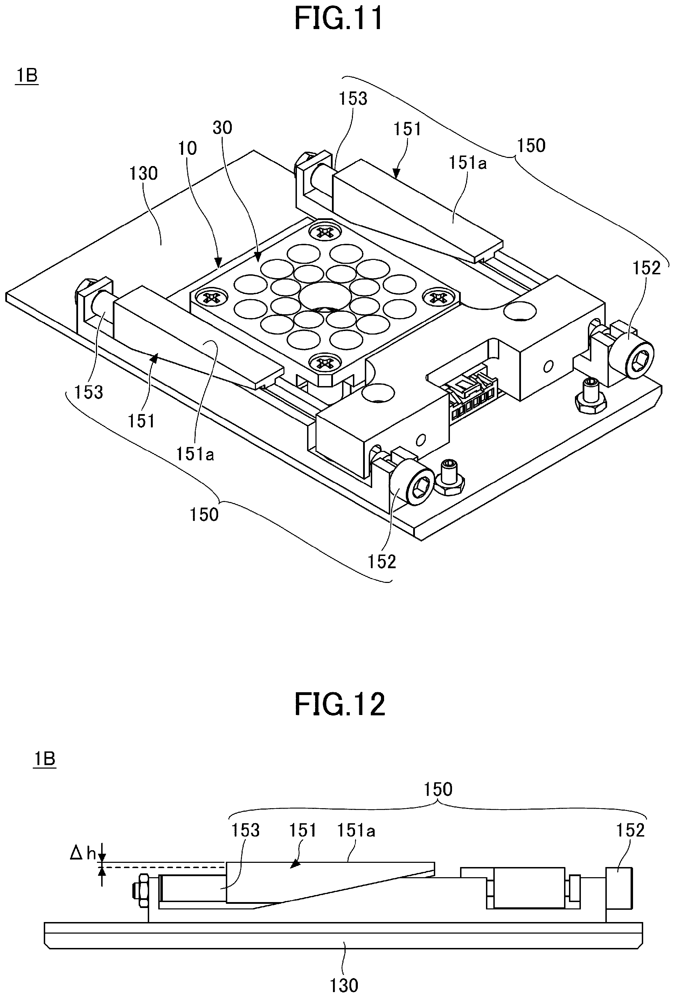

FIG. 11 is a perspective view (part 1) of a light source module according to a third modification of the first embodiment. FIG. 12 is a side view of the light source module according to the third modification of the first embodiment. As illustrated in FIG. 11, in a light source module 1 B, a substrate 10 on which a light source 20 and an optical member 30 are provided is fixed to a plate-shaped holding member 130. The substrate 10 can be fixed to the upper surface of the holding member 130 by, for example, a screw, an adhesive, or the like.

Two jack mechanisms 150 opposite to each other with the substrate 10 interposed therebetween in a top view are disposed on the holding member 130. Each of the jack mechanisms 150 includes a support member 151 having a upper surface 151a, a screw member 152, and an auxiliary spring member 153. The support member 151 is composed of, for example, a resin material, a metal material, or the like. The support member 151 is coupled to the screw member 152.

As illustrated in FIG. 12, the upper surface 151a of the support member 151 can move in a direction normal to the upper surface 151a by the rotation of the screw member 152. The amount of movement Δh illustrated in FIG. 12 indicates the amount of movement of the upper surface 151a of the support member 151 by the rotation of the screw member 152. A dashed line in FIG. 12 indicates the position of the upper surface 151a of the support member 151 in a state in which the upper surface 151a is lowered. A solid line in FIG. 12 indicates the position of the upper surface 151a of the support member 151 in a state in which the upper surface 151a is raised. The amount of movement Δh is, for example, 1 mm. The auxiliary spring member 153 applies a force that preloads the support member 151 toward the screw head of the screw member 152.

The light source module 1B including the jack mechanisms 150 can be easily fixed to a light source placement portion having a space where the light source module 1B can be disposed. For example, the light source module 1B is inserted into the space of the light source placement portion in a state in which the upper surface 151a of the support member 151 is lowered, and thereafter, the upper surface 151a of the support member 151 is raised by rotating the screw member 152, thereby allowing the light source module 1B to be fixed into the space of the light source placement portion.

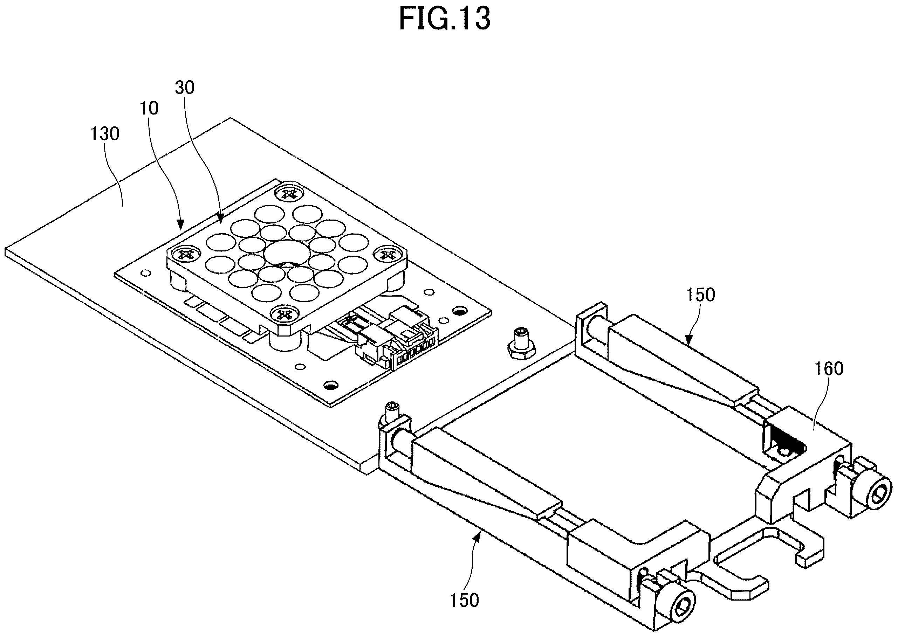

FIG. 13 is a perspective view (part 2) of the light source module according to the third modification of the first embodiment. As illustrated in FIG. 13, the jack mechanisms 150 may be provided separately from the holding member 130 and may be detachably attachable to the holding member 130 by a screw or the like. In the example illustrated in FIG. 13, the two jack mechanisms 150 are coupled to each other via a handle 160. The jack mechanisms 150 and the handle 160 may be integrated or separated.

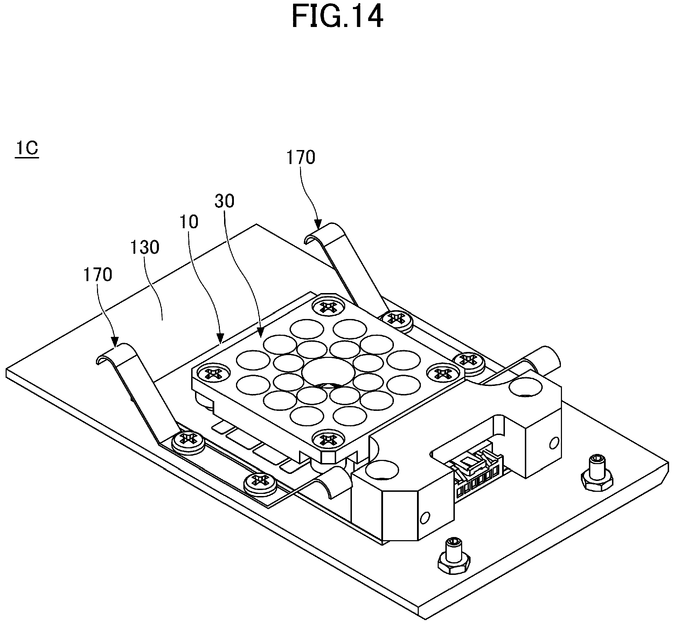

FIG. 14 is a perspective view of a light source module according to a fourth modification of the first embodiment. As illustrated in FIG. 14, a light source module 1C differs from the light source module 1B in that the light source module 1C includes spring members 170 instead of the jack mechanisms 150.

The spring members 170 are metal leaf springs. The spring members 170 are disposed on the holding member 130 so as to be opposite to each other with the substrate 10 interposed therebetween in a top view. The spring members 170 can be fixed to the upper surface of the holding member 130 by, for example, a screw. The number of the spring members 170 is not limited to two, and may be any number greater than or equal to one. Further, the shape of each of the spring members 170 is not limited to the shape illustrated in FIG. 14, and each of the spring members 170 may have any shape.

As described above, the light source module may include the spring members 170 instead of the jack mechanisms 150. The light source module 1C can be fixed into the space of the light source placement portion by the spring members 170.

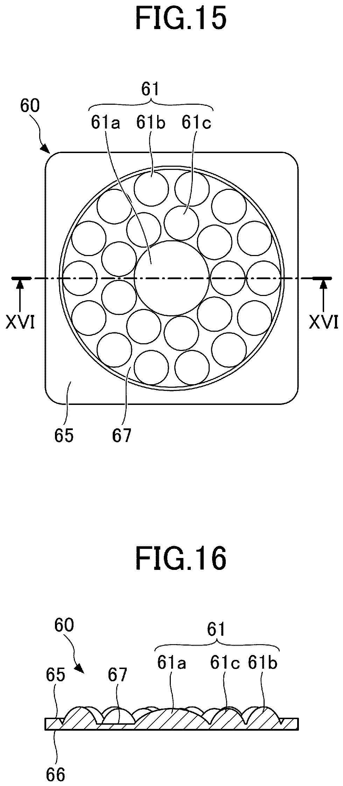

FIG. 15 is a top view of an optical member according to another example. FIG. 16 is a cross-sectional view of the optical member taken through line XVI-XVI of FIG. 15. The light source module 1 may include an optical member 60 instead of the optical member 30.

The optical member 60 includes a plurality of light controllers 61. In the example illustrated in FIG. 15 and FIG. 16, the optical member 60 has an upper surface 65 and a lower surface 66, and further has a recessed portion 67 that opens at the upper surface 65. The bottom surface of the recessed portion 67 is, for example, a flat surface. The bottom surface of the recessed portion 67 is, for example, parallel to the upper surface 65. The bottom surface of the recessed portion 67 is located at a position lower than the upper surface 65 and higher than the lower surface 66. The distance from the upper surface 65 to the bottom surface of the recessed portion 67 can be, for example, approximately one-half of the distance from the upper surface 65 to the lower surface 66. In a top view, the outer edge of the bottom surface of the recessed portion 67 has, for example, a circular shape.

The plurality of light controllers 61 are disposed on the bottom surface of the recessed portion 67. Accordingly, the thickness of the optical member 60 can be reduced. Further, a portion of the optical member 60 located outward of the recessed portion 67 is thicker than the recessed portion 67, and thus the rigidity of the optical member 60 can be secured.

The light controllers 61 are protrusions that protrude toward the side opposite to a light source 20. The shapes and the functions of the protrusions can be the same as those of the protrusions of the light controllers 51. Similar to the optical member 50, the optical member 60 can be formed of glass or a resin.

The plurality of light controllers 61 include a first light controller 61a and a plurality of second light controllers 61b. In the example illustrated in FIG. 15 and FIG. 16, the plurality of light controllers 61 further include a plurality of third light controllers 61c. For example, the first light controller 61a, the second light controllers 61b, and the third light controllers 61c can be arranged in the same manner as the first light controller 51a, the second light controllers 51b, and the third light controllers 51c. Even when the optical member 60 including the light controllers 61 is used, the light source module 1 as a whole can emit light having narrow light distribution.

The relationship among the first light controller 61a, the second light controllers 61b, and the third light controllers 61c in terms of height can be the same as that of the optical member 50. Further, the relationship among the first light controller 61a, the second light controllers 61b, and the third light controllers 61c in terms of width can be the same as that of the optical member 50. The optical member 60 may be applied to the light source module 1A.

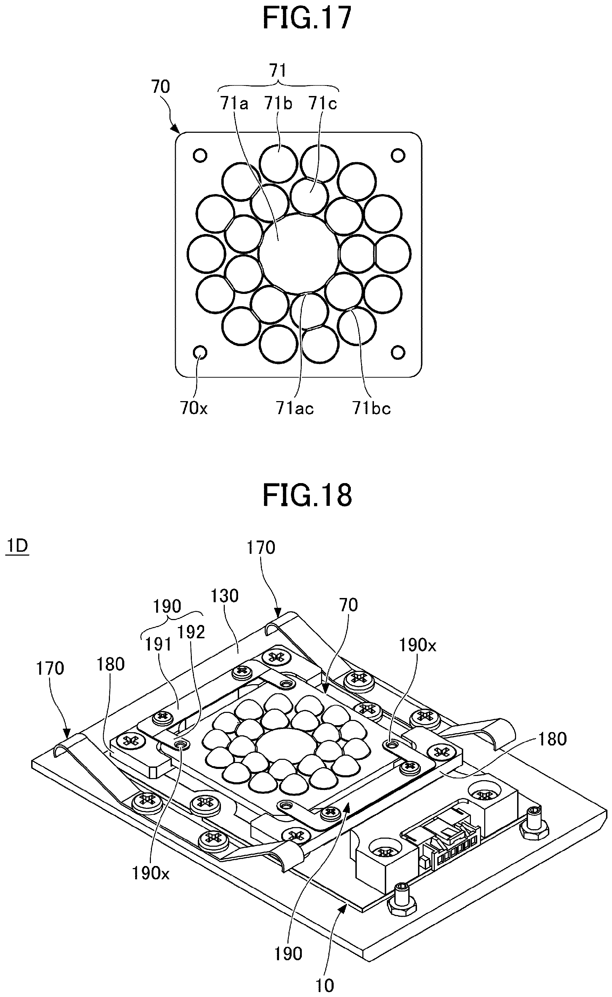

FIG. 17 is a top view of an optical member according to another example. The light source module 1 may include an optical member 70 instead of the optical member 30.

The optical member 70 includes a plurality of light controllers 71. In the example illustrated in FIG. 17, the optical member 70 has an upper surface and a lower surface, and the plurality of light controllers 71 are disposed on the upper surface of the optical member 70. The light controllers 71 are protrusions that protrude toward the side opposite to a light source 20. The shapes and the functions of the protrusions can be the same as those of the protrusions of the light controllers 51. Similar to the optical member 50, the optical member 70 can be formed of glass or a resin.

The plurality of light controllers 71 include a first light controller 71a and a plurality of second light controllers 71b. In the example illustrated in FIG. 17, the plurality of light controllers 71 further include a plurality of third light controllers 71c. For example, the first light controller 71a, the second light controllers 71b, and the third light controllers 71c can be arranged in the same manner as the first light controller 51a, the second light controllers 51b, and the third light controllers 51c. Even when the optical member 70 including the light controllers 71 is used, the light source module 1 as a whole can emit light having narrow light distribution.

In the light controllers 71, the first light controller 71a is connected to an adjacent third light controller 71c via a connection portion 71ac in a top view. The connection portion 71ac is a portion where a portion of the outer edge of the first light controller 71a and a portion of the outer edge of the adjacent third light controller 71c overlap each other. Further, some of the second light controllers 71b are connected to respective adjacent third light controllers 71c via connection portions 71bc in a top view. Each of the connection portions 71bc is a portion where a portion of the outer edge of a second light controller 71b and a portion of the outer edge of an adjacent third light controller 71c overlap each other. With such a configuration, the density of the arrangement of the light controllers can be increased, and thus the size of the light source module can be reduced.

The relationship among the first light controller 71a, the second light controllers 71b, and the third light controllers 71c in terms of height can be the same as that of the optical member 50. Further, the relationship among the first light controller 71a, the second light controllers 71b, and the third light controllers 71c in terms of width can be the same as that of the optical member 50.

The optical member 70 may have a plurality of recesses 70x that open in outer peripheral portions of the upper surface. In the example of FIG. 17, the optical member 70 has four recesses 70x in the vicinity of the corners of the upper surface. Each of the recesses 70x may have, for example, a triangular pyramid shape. The recesses 70x can be used, for example, when the optical member 70 is held on the substrate 10 as illustrated in FIG. 18.

FIG. 18 is a perspective view of a light source module according to a fifth modification of the first embodiment. As illustrated in FIG. 18, a light source module 1D uses the optical member 70 instead of the optical member 30. The light source module 1D includes pedestal portions 180 and fixing members 190. The pedestal portions 180 are disposed on the uppers surface of the substrate 10 so as to be opposite to each other with the optical member 70 interposed therebetween, and the fixing members 190 are disposed on the pedestal portions 180.

The fixing members 190 are, for example, metal plates. Each of the fixing members 190 includes a fixing portion 191 and spring portions 192. The fixing portion 191 extends in the short-side direction of the upper surface of the substrate 10 and is fixed to a corresponding pedestal portion 180. The spring portions 192 extends from both ends of the fixing portion 191 onto the upper surface of the optical member 70. The fixing portion 191 can be fixed to the upper surface of the corresponding pedestal portion 180 by, for example, a screw.

The tip on the optical member 70 side of each of the spring portions 192 is provided with a protrusion 190x protruding toward the upper surface of the optical member 70. The protrusion 190x is fitted into a corresponding recess 70x provided in the upper surface of the optical member 70. The optical member 70 is preloaded toward the substrate 10 by the fixing members 190, and the optical member 70 is held on the substrate 10. Similar to the optical member 70, the other optical members may have recesses. With such a configuration, the same fixing method as that of the optical member 70 can be employed. The optical member 70 may be fixed to the substrate 10 by, for example, an adhesive. As the material of the adhesive, a material having light resistance to ultraviolet light is preferably used.

### Second Embodiment

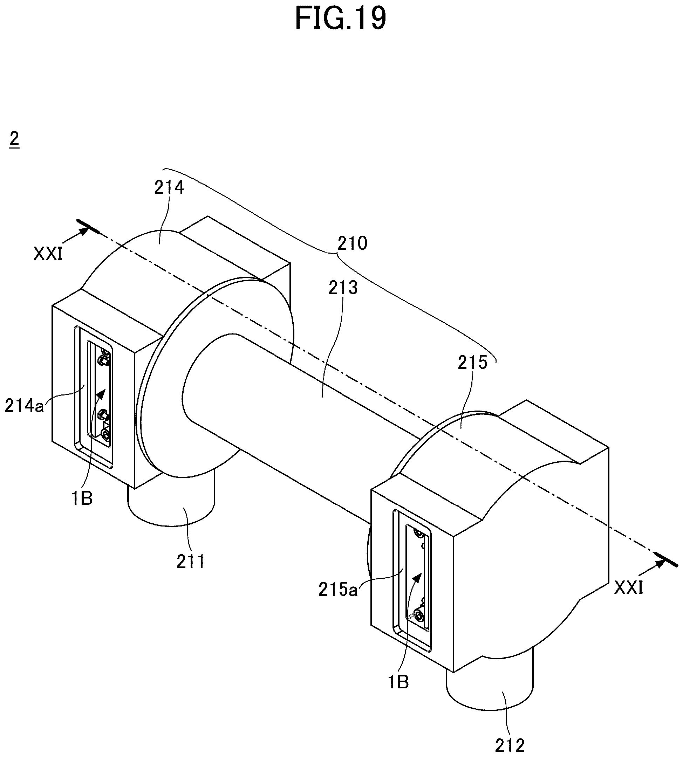

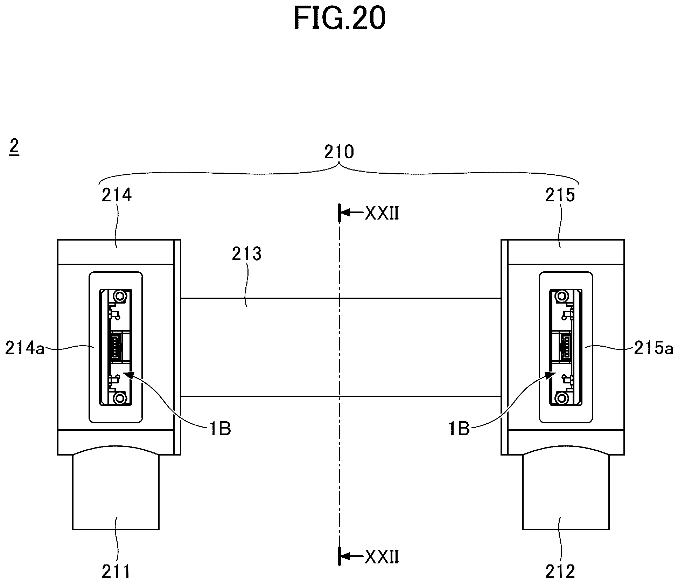

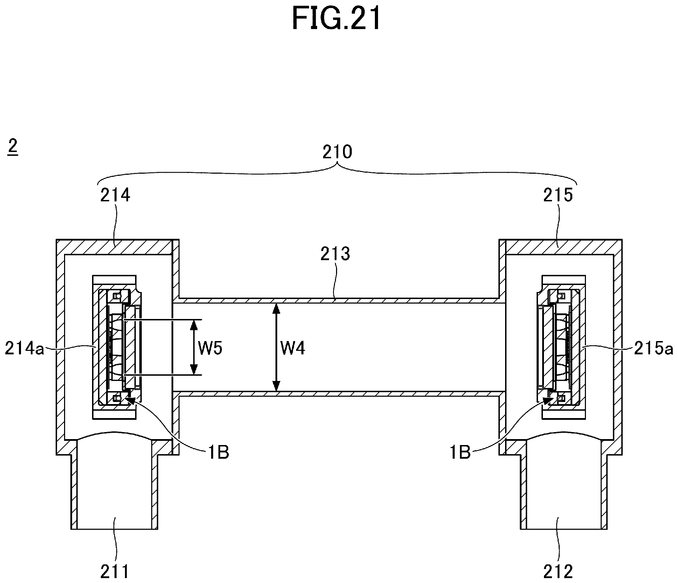

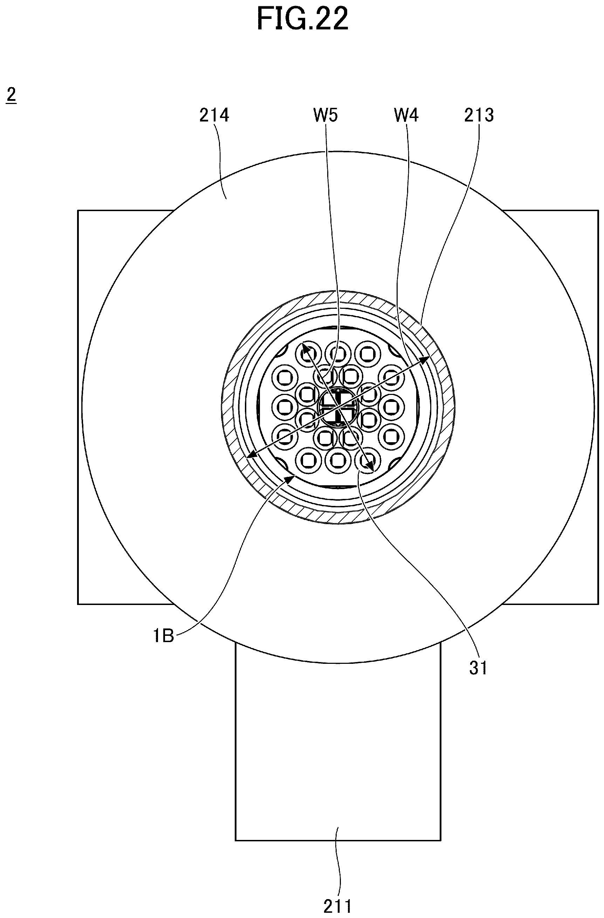

FIG. 19 is a perspective view of an ultraviolet light fluid treatment device according to a second embodiment. FIG. 20 is a side view of the ultraviolet light fluid treatment device according to the second embodiment. FIG. 21 is a cross-sectional view of the ultraviolet light fluid treatment device taken through line XXI-XXI of FIG. 19. FIG. 22 is a cross-sectional view of the ultraviolet light fluid treatment device taken through line XXII-XXII of FIG. 20.

As illustrated in FIG. 19 to FIG. 22, an ultraviolet light fluid treatment device includes a flow channel tube 210 in which fluid flows and two light source modules 1B. The number of light source modules 1B may be one or three or more. Instead of the light source modules 1B, light source modules 1C or light source modules 1D may be used.

The flow channel tube 210 includes an inlet 211 and an outlet 212 of the fluid, a flow channel 213 in which the fluid flows, a first connection portion 214 connecting the inlet 211 and the flow channel 213, and a second connection portion 215 connecting the outlet 212 and the flow channel 213. The fluid is introduced from the inlet 211, flows through the flow channel 213, and flows out of the outlet 212. The direction of flow of the fluid may be an opposite direction.

One of the two light source modules 1B is disposed in the first connection portion 214 such that an optical member 30 faces the flow channel 213. The one of the two light source modules 1B is inserted into a light source placement portion 214a provided in the first connection portion 214, and is fixed to the light source placement portion 214a by jack mechanisms 150 (see FIG. 11).

The other light source module 1B is disposed in the second connection portion 215 such that an optical member 30 faces the flow channel 213. The two light source modules 1B face each other with the flow channel 213 interposed therebetween. The other light source module 1B is inserted into a light source placement portion 215a provided in the second connection portion 215, and is fixed to the light source placement portion 215a by jack mechanisms 150 (see FIG. 11).

The lower surface of a holding member 130 (see FIG. 11) constituting part of the one of the two light source modules 1B can make surface contact with the inner wall of the light source placement portion 214a. Further, the lower surface of a holding member 130 (see FIG. 11) constituting part of the other light source module 1B can make surface contact with the inner wall of the light source placement portion 215a. Accordingly, heat generated during the operation of the light source modules can be released to the fluid flowing around the light source placement portions.

The flow channel 213 extends in a direction normal to an upper surface 10a of a substrate 10 of the one of the two light source modules 1B. Further, the flow channel 213 extends in a direction normal to an upper surface 10a of a substrate 10 of the other light source module 1B. The light source modules 1B can irradiate the flow channel 213 with ultraviolet light emitted from the optical members 30.

As described above, in the example illustrated in FIG. 19 to FIG. 22, the ultraviolet light fluid treatment device 2 can irradiate the fluid flowing through the flow channel 213 with the ultraviolet light from the two light source modules 1B. Accordingly, the ultraviolet light fluid treatment device 2 can treat the fluid flowing through the flow channel 213 by irradiating the fluid with the ultraviolet light, such that the number of bacteria and viruses in the fluid can be reduced after the treatment as compared to before the treatment, for example. Examples of the fluid include liquid, gas, and the like.

As illustrated in FIG. 21 and FIG. 22, a width W4 of the flow channel 213 is preferably 1.0 times or more and 2.0 times or less the maximum width W5 of a region where a plurality of light controllers 31 are disposed in a top view. The maximum width W5 is defined by the length of the longest straight line that can be drawn from the outer edge of one light controller 31 to the outer edge of another light controller 31 among the plurality of light controllers 31 in a top view. When the width W4 is 1.0 times or more the width W5, of light emitted from a peripheral portion of each of the optical members 30, the amount of light that is attenuated by striking the inner wall of the flow channel 213 can be reduced. As a result, the ultraviolet light fluid treatment device 2 having high processing efficiency can be achieved. Further, when the width W4 is 2.0 times or less the width W5, the fluid flowing near the inner wall of the flow channel 213 can also be irradiated with the light emitted from the peripheral portion of each of the optical members 30.

Light emitted from peripheral portions located above a peripheral region R2 and an intermediate region R3 of each of the optical members 30 has narrower light distribution than light emitted from the center located above a central region R1. The light emitted from the peripheral portions located above the peripheral region R2 and the intermediate region R3 of each of the optical members 30 has a light distribution angle of, for example, 10° or more and 60° or less. The light emitted from the center located above the central region R1 has a light distribution angle of, for example, 110° or less.

According to one embodiment of the present disclosure, a light source module including a novel optical member that corresponds to the arrangement of light sources can be provided.

Although embodiments have been described in detail above, the above-described embodiments are non-limiting examples, and various modifications and substitutions can be made to the above-described embodiments without departing from the scope described in the claims.

### DESCRIPTION OF THE REFERENCE NUMERALS

1, 1A, 1B, 1C, 1D light source module
10 substrate
10a upper surface
11 wiring
12 connector
20 light source
20a first light emitting part
20b second light emitting part
20c third light emitting part
30, 50, 60, 70 optical member
31, 51, 61, 71 light controller
31a, 51a, 61a, 71a first light controller
31b, 51b, 61b, 71b second light controller
31c, 51c, 61c, 71c third light controller
41 spacer
42 screw
65 upper surface
66 lower surface
67 recessed portion
70x recessed portion
130 holding member
150 jack mechanism
151 support member
151a upper surface
152 screw member
153 auxiliary spring member
160 handle
170 spring member
180 pedestal portion
190 fixing member
190x protrusion
191 fixing portion
192 spring member

## Claims

1. A light source module comprising:
a substrate having an upper surface;
a light source disposed on the upper surface of the substrate; and
an optical member including a plurality of light controllers and disposed above the substrate with the light source interposed therebetween, wherein
the light source includes a plurality of first light emitting parts and a plurality of second light emitting parts,
the plurality of light controllers include a first light controller and a plurality of second light controllers,
the first light controller overlaps the plurality of first light emitting parts in a top view, and
each of the plurality of second light controllers overlaps a corresponding one of the plurality of second light emitting parts in the top view.

2. The light source module according to claim 1, wherein
the plurality of first light emitting parts are disposed in a central region and the plurality of second light emitting parts are disposed in a peripheral region located outward of the central region in the top view, and
the first light controller is disposed in the central region and the plurality of second light controllers are disposed in the peripheral region in the top view.

3. The light source module according to claim 2, wherein
the light source further includes a plurality of third light emitting parts disposed in an intermediate region located between the central region and the peripheral region in the top view,
the plurality of light controllers further include a plurality of third light controllers disposed in the intermediate region in the top view, and
each of the plurality of third light controllers overlaps a corresponding one of the plurality of third light emitting parts in the top view.

4. The light source module according to claim 1, wherein a distance between adjacent ones of the plurality of first light emitting parts is smaller than a distance between adjacent ones of the plurality of second light emitting parts.

5. The light source module according to claim 4, wherein
the light source further includes a plurality of third light emitting parts,
the plurality of light controllers further include a plurality of third light controllers,
the plurality of third light controllers overlap the plurality of third light emitting parts in the top view, and
a distance between adjacent ones of the plurality of first light emitting parts is smaller than a distance between adjacent ones of the plurality of third light emitting parts.

6. The light source module according to any one of claims 1 to 5, wherein at least one of the plurality of second light controllers has a width smaller than a width of the first light controller in the top view.

7. The light source module according to claim 3 or 5, wherein a width of each of the plurality of second light controllers is greater than a width of each of the plurality of third light controllers in the top view.

8. The light source module according to claim 3 or 5, wherein a width of each of the plurality of second light controllers is equal to a width of each of the plurality of third light controllers in the top view.

9. The light source module according to any one of claims 1 to 8, wherein
each of the plurality of light controllers is an opening penetrating the optical member in a direction normal to the upper surface of the substrate, and
an opening area of the opening is smallest on a light source side thereof and the opening area of the opening increases as a distance from the light source increases

10. The light source module according to any one of claims 1 to 8, wherein
each of the plurality of light controllers is a protrusion protruding toward a side opposite to the light source, and
the protrusion is thickest at a center thereof and becomes thinner as a distance from the center increases in the top view.

11. The light source module according to any one of claims 1 to 10, wherein ultraviolet light is emitted from the optical member.

12. An ultraviolet light fluid treatment device comprising:
a flow channel in which fluid flows; and
the light source module of claim 11 configured to irradiate the flow channel with the ultraviolet light emitted from the optical member.

13. The ultraviolet light fluid treatment device according to claim 12, wherein a width of the flow channel is 1.0 times or more and 2.0 times or less a maximum width of a region where the plurality of light controllers are disposed in the top view.

14. The ultraviolet light fluid treatment device according to claim 12 or 13, wherein a light distribution angle of light emitted from each of the plurality of second light controllers is smaller than a light distribution angle of light emitted from the first light controller.

15. The ultraviolet light fluid treatment device according to any one of claims 12 to 14, further comprising:
an inlet of the fluid; and
a first connection portion connecting the inlet and the flow channel, wherein
the light source module is disposed in the first connection portion, and
the flow channel extends in a direction normal to the upper surface of the substrate.
